# EUROPEAN PATENT SPECIFICATION

(11) **EP 1 341 561 B1**
(45) Date of publication and mention of the grant of the patent: **07.02.2007**
(21) Application number: 01270350.0
(22) Date of filing: 11.12.2001
(51) Int. Cl.: A61L 15/42

(54) **DRESSINGS FOR THE TREATMENT OF EXUDING WOUNDS**
VERBÄNDE ZUR BEHANDLUNG VON EXSUDIERENDEN WUNDEN
PANSEMENT POUR TRAITEMENT DE PLAIES SUINTANTES

(30) Priority: 12.12.2000 GB 0030308
(43) Date of publication of application: 10.09.2003
(73) Proprietor: JOHNSON & JOHNSON MEDICAL LIMITED., Edinburgh EH2 4NH (GB)
(72) Inventor: SILCOCK, Derek, Skipton, North Yorkshire BD23 1QP (GB); MARSDEN, Donald, Christopher, Skipton, North Yorkshire BD23 2th (GB)
(74) Representative: James, Anthony Christopher W.P.
(86) International application number: PCT/GB2001/005466
(87) International publication number: WO 2002/047737

(56) References cited:
- EP-A- 0 612 520
- WO-A-92/02199
- WO-A-98/17328
- US-A- 4 062 451
- US-A- 4 909 244
- US-A- 6 153 215
- US-A- 6 160 200

## Description

The present invention relates to wound dressings, and in particular to a new layered wound dressing structure.

It is known that the maintenance of a moist wound environment promotes the healing of wounds, especially bums and chronic wounds such as ulcers. However, it is also desirable to avoid excessive moisture or pooling of wound exudate on the wound, since liquid exudate causes maceration of skin adjacent to the wound and other difficulties. Furthermore, liquid exudate can leak from the wound site and contaminate clothes or bedding.

In practice, it is difficult to maintain the desired moisture level at the wound site because the rate of wound fluid production varies from wound to wound, and over time for any single wound. This can necessitate frequent dressing changes and a range of dressing types to treat different wounds. For example, infected wounds generally produce substantially more exudate than non-infected wounds. Surgical wounds have an acute inflammatory phase of a few days during which discharge is significant, after which the rate of exudate production can be expected to fall sharply.

Studies have been carried out on the pH of the wound environment during wound healing. Whilst it is no simple matter to determine the actual pH at a wound site, it appears that the pH of chronic wounds is neutral or slightly alkaline, whereas the pH of intact skin is slightly acidic (pH 4 or 5). It also appears that prolonged acidification of chronic wound surfaces using acid buffered solutions increases the healing rate. However, it is not practical or desirable to irrigate chronic wounds with acid-buffered solutions in normal medical practice.

US-A-4813942 describes a debridement wound dressing comprising a hydrocolloid wound contacting layer containing 35% to 50% w/w of pectin or other hydrocolloids capable of reducing the pH of the wound/dressing interface to 4.8-6.5. The debridement dressing is left in place for 24-48 hours, and is then replaced by a regeneration wound dressing that provides near-neutral pH at the wound surface.

US-A-6160200 describes absorbent articles, in particular diapers and incontinence pads, comprising a directionally preferential waste passage member. The waste passage member includes a soluble material capable of dissolving when contacted with bodily exudates, so as to permit the bodily exudates to pass through the waste passage member in a direction generally away from the wearer's skin. For example, the waste passage member may comprise a pH-sensitive material that dissolves in the presence of bodily exudates. The waste passage member may be coated by an adhesive to hold the waste passage member dose to the wearer's skin. The adhesive may be a gel adhesive. The waste passage member may further comprise a skin care composition which may be used as, with, or In place of the body adhering composition. The skin care composition is typically a lotion, and it may comprise a pH control agent such as citric acid.

Antimicrobial wound dressings containing antibiotics or other antimicrobial therapeutic agents for the treatment of infected wounds are known. However, such dressings are not indicated for prophylactic use on wounds that are not obviously infected, because of concerns about microbial resistance and risks of unnecessary medication. A need exists for new wound dressings that address these concerns.

It is an object of the present invention to provide structures for use as or in improved multilayer dressings for the treatment of a wide range of wounds.

It is a further object of the present invention to provide layered wound dressing structures that can maintain a lowered pH at the surface of a wound to assist wound healing.

It is a further object of the present invention to provide layered wound dressing structures that can release active therapeutic agents selectively into exuding wounds.

The present invention provides a layered wound dressing material comprising: a wound facing hydrogel layer; and a barrier layer, wherein the barrier layer comprises a pH-sensitive material that is substantially insoluble in water at 25°C and pH4, but substantially soluble in water at 25°C and pH8, and wherein the hydrogel layer comprises a weak water-soluble acid buffer system.

Preferably, the barrier layer is in contact with the wound facing hydrogel layer, and it may be bonded thereto chemically or physically. The hydrogel layer comprises a weak water-soluble acid buffer system. That is to say, a mixture of a weak water-soluble acid and its conjugate base.

In use, the hydrogel layer absorbs and buffers wound exudate to provide a moist and preferably slightly acidified wound environment suitable for healing. As the hydrogel layer becomes saturated with wound exudate, which is neutral or slightly alkaline, its pH rises until it reaches a range at which the pH-sensitive material in the barrier layer starts to dissolve. This breakdown of the pH-sensitive material increases the liquid permeability of the barrier layer and thereby allows the excess exudate to escape through the barrier layer, preferably into an absorbent layer. Thus, the layered material is suitable for the treatment of wounds having a range of different exudation rates, and for extended periods.

Preferably, the layered material according to the invention further comprises an absorbent layer separated from the wound facing hydrogel layer by the barrier layer. The area of the optional absorbent layer is typically in the range of from 1cm² to 200cm², more preferably from 4cm² to 100cm².

The optional absorbent layer may be any of the layers conventionally used for absorbing wound fluids, serum or blood in the wound healing art, including gauzes, nonwoven fabrics, superabsorbents, hydrogels and mixtures thereof. Preferably, the absorbent layer comprises a layer of absorbent foam, such as an open celled hydrophilic polyurethane foam prepared in accordance with EP-A-0541391, the entire content of which is expressly incorporated herein by reference. In other embodiments, the absorbent layer may be a nonwoven fibrous web, for example a carded web of viscose staple fibers. The basis weight of the absorbent layer may be in the range of 50-500g/m², such as 100-400g/m². The uncompressed thickness of the absorbent layer may be in the range of from 0.5mm to 10mm, such as 1mm to 4mm. The free (uncompressed) liquid absorbency measured for physiological saline may be in the range of 5 to 30 g/g at 25°C.

Preferably the layered wound dressing material according to the invention further comprises a reservoir of antimicrobial material over the barrier layer opposite the hydrogel layer. The reservoir is preferably a layer of antimicrobial-containing material. For example, the antimicrobial layer may be coated over the barrier layer, or it may be dispersed in the absorbent layer. The antimicrobial is only released into the wound after the barrier layer is dissolved by the action of wound exudate. This enables selective release of the antimicrobial only into exuding wounds, such as infected wounds. Suitable antimicrobials include antibiotics, silver salts and chlorhexidine. Preferred amounts of the antimicrobials are from 0.01 to 100 mg/cm² of the barrier layer, more preferably from 0.1 to 10 mg/cm².

Preferably the layered wound dressing material according to the invention further comprises a liquid-impermeable backing layer over the barrier layer and the absorbent layer and antimicrobial reservoir (where present) opposite the hydrogel layer. The backing layer supports the barrier layer and the intermediate absorbent layer and antimicrobial reservoir (where present) and preferably provides a barrier to passage of microorganisms through the dressing. The backing layer may extend beyond at least one edge of the absorbent layer to provide an adhesive-coated margin adjacent to the said edge for adhering the dressing to a surface, such as to the skin of a patient adjacent to the wound being treated. An adhesive-coated margin may extend around all sides of the absorbent layer, so that the dressing is a so-called island dressing. However, it is not necessary for there to be any adhesive-coated margin.

Preferably, the backing layer is substantially liquid-impermeable. The backing layer is preferably semipermeable. That is to say, the backing layer is preferably permeable to water vapour, but not permeable to liquid water or wound exudate. Preferably, the backing layer is also microorganism-impermeable. Suitable continuous conformable backing layers will preferably have a moisture vapor transmission rate (MVTR) of the backing layer alone of 300 to 5000 g/m²/24hrs, preferably 500 to 2000 g/m²/24hrs at 37.5 °C at 100% to 10% relative humidity difference. The backing layer thickness is preferably in the range of 10 to 1000 micrometers, more preferably 100 to 500 micrometers.

The MVTR of the dressing according to the present invention as a whole is lower than that of the backing layer alone, because the barrier layer partially obstructs moisture transfer through the dressing. Preferably, the MVTR of the dressing (measured across the island portion of the dressing) is from 20% to 80% of the MVTR of the backing layer alone, more preferably from 20% to 60% thereof, and most preferably about 40% thereof. It has been found that such moisture vapor transmission rates allow the wound under the dressing to heal under moist conditions without causing the skin surrounding the wound to macerate.

Suitable polymers for forming the backing layer include polyurethanes and poly alkoxyalkyl acrylates and methacrylates such as those disclosed in GB-A-1280631. Preferably, the backing layer comprises a continuous layer of a high density blocked polyurethane foam that is predominantly dosed-cell. A suitable backing layer material is the polyurethane film available under the Registered Trade Mark ESTANE 5714F.

The adhesive layer (where present) should be moisture vapor transmitting and/or patterned to allow passage of water vapor therethrough. The adhesive layer is preferably a continuous moisture vapor transmitting, pressure-sensitive adhesive layer of the type conventionally used for island-type wound dressings, for example, a pressure sensitive adhesive based on acrylate ester copolymers, polyvinyl ethyl ether and polyurethane as described for example in GB-A-1280631- The basis weight of the adhesive layer is preferably 20 to 250 g/m², and more preferably 50 to 150 g/m². Polyurethane-based pressure sensitive adhesives are preferred.

Preferably, the hydrogel layer has a dry basis weight of from 10 to 200g/m², more preferably from 10 to 100g/m², and most preferably from 10 to 50g/m².

The term "hydrogel layer" refers to layers that form a gel with water under physiological conditions. Such hydrogel layers can be formed by the inclusion of medically acceptable macromolecular materials that have the ability to swell and absorb fluid while maintaining a strong integral structure. Preferably, the hydrogel composition forms a gel that is substantially insoluble in water under physiological conditions, whereby the hydrogel is not washed away by the wound fluid. The hydrogel may be a biopolymer, and/or it may be bioabsorbable. That is to say, it may undergo gradual resorption *in vivo.*

Exemplary insoluble gels include certain cross-linked polyacrylate gels, calcium alginate gels, cross-linked hyaluronate gels, wherein the hydrogel layer comprises a hydrogel material selected from gels formed from vinyl alcohols, vinyl esters, vinyl ethers and carboxy vinyl monomers, meth(acrylic) acid, acrylamide. N-vinyl pyrrolidone, acylamidopropane sulphonic acid, PLURONIC (Registered Trade Mark) (block polyethylene glycol, block polypropylene glycol) polystyrene-, maleic acid, NN-dimethylacrylamide diacetone acrylamide, acryloyl morpholine, -and mixtures thereof. Preferably, the gel adheres strongly to the surface of the barrier layer material to resist washing off by wound fluid. In certain embodiments the gel may be chemically bonded to the surface of the barrier layer.

Preferably, the hydrogel layer comprises a hydrogel material selected from polyurethane gets, biopolymer gels, carboxymethyl cellulose gels, hydroxyethyl cellulose gels, hydroxy propyl methyl cellulose, modified acrylamide and mixtures thereof. Suitable biopolymer gels include alginates, pectins, galactomannans, chitosan, gelatin, hyaluronates and mixtures thereof. Some of these biopolymer materials also promote wound healing.

Preferably, the gels are cross-linked, and the cross-linking may be either covalent or ionic. It will be appreciated that the degree of cross linking will influence the physical properties and rate of resorption of the hydrogel layer *in vivo.*

Preferably, the hydrogel material further comprises from 5 to 50% by weight on a dry weight basis of one or more humectants such as glycerol

Preferably, the hydrogel layer comprises a hydrogel material of the kind described in WO00/07638, the entire content of which is incorporated herein by reference.

The hydrogel layer may additionally comprise one or more active therapeutic or antimicrobial agents. Suitable therapeutic agents include growth factors, analgesics, local anaesthetics and steroids. Suitable antimicrobial agents include antiseptics such as silver compounds and chlorhexidine, and antibiotics. The therapeutic or antimicrobial agents are usually added in an amount of from 0.01% to 5% by weight, based on the dry weight of the hydrogel layer.

The hydrogel layer may be continuous or discontinuous. The hydrogel layer may be applied by spraying or by a printing or transfer process.

The hydrogel material may be inherently acidic, for example it may comprise a polymeric gel-forming acid such as hyaluronic acid, alginic acid, pectic acid, or a polymer or copolymer of an acrylic acid. The layered wound dressing material according to the present invention further comprises a weak, water-soluble acid buffer system dissolved or dispersed in the hydrogel.

Preferably, the acid buffer system is present in an amount of at least 0.1 mmol/g, preferably at least 0.2 mmol/g, more preferably at least 0.3 mmol/g. The upper limit of the amount of acid buffer system is preferably about 5.0 mmol/g, more preferably about 2.0 mmol/g. The amount of acid buffer system is calculated as the total weight of water-soluble weak acid plus the total weight of water-soluble conjugate base. The amount is based on the dry weight of the hydrogel material.

Suitably, a 10%w/v dispersion (dry weight basis) of the hydrogel material in water provides an equilibrium pH at 25°C of from 4.5 to 6.5.

The acid buffer system is a combination of one or more physiologically acceptable weak water-soluble acids with one or more physiologically acceptable water-soluble conjugate bases of weak acids. Preferably, the conjugate base is a conjugate base of the weak acid present In the system. Preferably, the acid components of the water-soluble acid buffer system have a pKₐ in the range of from 3.5 to 6.5.

The presence of a buffer system comprising both a weak acid and a conjugate base enables the materials according to the present invention to maintain a stable pH in a wound environment over an extended period of time during which gradual neutralisation of the acid is taking place, in accordance with well known physicochemical principles. For good buffering performance, the molar ratio of add to base in the buffer system should be in the range of from 1:10 to 10:1, preferably from 1:5 to 5:1, and more preferably from 2:5 to 5:2.

Preferably, the water-soluble acid has a low vapour pressure, and more preferably it is solid when anhydrous at room temperature. Preferably, the water-soluble acid has a solubility of at least 10 mg/ml at 25°C in water, more preferably at least 20 mg/ml, and still more preferably at least 50 mg/ml.

Wound dressings based on macromolecular acids such as alginic acid, certain viscose fibres, or oxidized regenerated cellulose are known. However, these macromolecular acids have limited solubility, limited buffering capacity and provide little ability to control the pH at a wound surface. The water-soluble acid according to the present invention is not a macromolecular substance.

The weak, water-soluble acid buffer system provides a mildly acidic (preferably pH 3.5 to 5.5) environment at the wound surface. Accordingly, the water-soluble acid component of the buffer system preferably has a pKₐ in the range of from 3 to 8, more preferably from 3.5 to 6.5. Preferably, the conjugate base is the conjugate base of the water-soluble acid present in the material, for example a salt of the weak water-soluble acid, more preferably a sodium salt thereof.

Preferably, the weak water-soluble acid and conjugate base are selected from the group consisting of water-soluble organic carboxylic acids and dihydrogen phosphate salts, and mixtures and conjugate bases thereof. The preferred organic carboxylic acids include C₂-C₆ alkanoic acids, benzoic acid, substituted benzoic acids, citric acid, tartaric acid, malic acid, lactic acid, succinic acid, and ascorbic acid. It goes without saying that the water-soluble acid and conjugate base used in the materials according to the present invention should be fully biologically and pharmaceutically acceptable for topical application to a wound.

Preferably, the water-soluble acid and the conjugate base are both nonvolatile. More preferably, both materials are solids when pure and anhydrous at 25°C. The most preferred buffer system is hydrogen phosphate/dihydrogen phosphate. Other highly preferred systems are citric acid/citrate and lactic acid/lactate. Sodium salts or sodium/potassium mixed salts of the conjugate bases are preferred.

The concentration of the weak, water-soluble acid in the hydrogel layer of the material according to the present invention will depend on the particular application, the nature of the wound to which the material is to be applied, and the desired rate of breakdown of the hydrogel and barrier layers in use.

The acid buffering capacity of the hydrogel layer according to the present invention can be determined, for example, by slurrying 1 g of the hydrogel layer in 10 mls of deionised water and measuring the amount of alkali (sodium hydroxide) needed to raise the pH of the slurry to 10. The acid buffering capacity of the material is preferably at least 0.05 mmol/g, more preferably at least 0.1 mmol/g, and most preferably at least 0.2 mmol/g, based on the dry weight of the hydrogel layer

The layered material according to the present invention preferably provides a pH at the wound site in use in the range of from 3.5 to 6.5, more preferably from 4.5 to 6.5. As more exudate is produced, it neutralises the acid present in the hydrogel and increases the pH of the hydrogel layer until it reaches a range at which dissolution of the pH sensitive material takes place.

The pH-sensitive material is substantially insoluble in water at 25°C and pH 4 and substantially soluble in water at 25°C and pH 8. Preferably, the polymer becomes soluble with increasing pH at a pH in the range of 5 to 7, more preferably 5.5 to 6.5. In this context the term "soluble" preferably denotes an equilibrium solubility of the material greater than 1%w/w in water at 25°C. Particularly suitable are film-forming polymers and mixtures, such as those used to provide enteric coatings on orally administered medicaments.

Preferably, the pH-sensitive material comprises a polymer selected from the group consisting of cellulose derivatives, starch derivatives, pectins, polyacrylates, polyvinyl acetate phthalate, and mixtures thereof.

Preferred cellulose derivatives are selected from cellulose acetate phthalate, cellulose acetate trimellitate, hydroxypropyl methyl cellulose, hydroxypropyl methyl cellulose acetate succinate, carboxymethyl ethyl cellulose, oxidised regenerated cellulose, and mixtures thereof.

Preferred polyacrylates are selected from the copolymers of methacrylic acid with methyl methacrylate. Particularly preferred are various copolymers of this type sold under the Registered Trade Mark EUDRAGIT. By varying the ratio of methacrylic acid to methyl methacrylate it is possible to control the pH at which these copolymers dissolve in order to optimise the properties of the material.

The barrier layer is preferably substantially liquid-impermeable before dissolution of the pH-sensitive material. In certain embodiments the barrier layer comprises a substantially continuous film comprising the pH-sensitive material, and preferably consisting essentially of the pH-sensitive material, optionally mixed with plasticisers, fillers, indicator substances, or medicating agents.

In other preferred embodiments, the barrier layer comprises a liquid-permeable support layer having the pH-sensitive material applied thereto, preferably in occlusive fashion to reduce the liquid permeability of the layer until the pH-sensitive material is dissolved- For example, the support layer may comprise a water-insoluble perforated film or nonwoven or woven fabric having the pH-sensitive material coated thereon in partially or completely occlusive fashion.

The present invention further provides a wound dressing comprising a layered structure according to the present invention. Preferably, the wound dressing further comprises an absorbent layer and/or a reservoir of antimicrobial and/or a backing layer and/or any other preferred features as described above in connection with preferred embodiments of the layered structures according to the invention.

Preferably, a liquid-absorbent layer is provided on the wound contacting side of the wound dressing over the barrier layer. A further liquid-permeable wound contacting top sheet may also be provided. The barrier layer in the wound dressing according to the present invention forms part of a layered wound dressing material according to the present invention as described above.

Preferably, the wound dressing according to the invention further comprises one or more protective cover sheets over the hydrogel layer and any exposed adhesive. For example, the cover sheets may comprise one or more release-coated paper cover sheets. Preferably, the dressing is sterile and packaged in a microorganism-impermeable container.

An embodiment of the present invention will now be described further, by way of example, with reference to the accompanying drawings, in which:
Figure 1 shows a perspective view of the lower (wound contacting) surface of a wound dressing according to the invention with the barrier layer and the hydrogel layer partially cut away; and
Figure 2 shows a partial transverse cross section (not to scale) through the island region of the dressing of Figure 1.

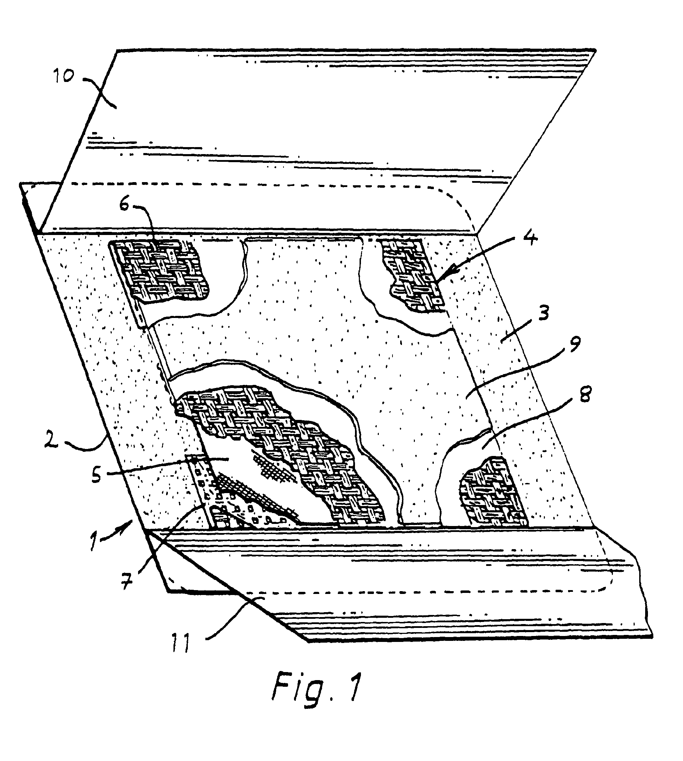

Referring to Figure 1, the wound dressing 1 is an island-type self-adhesive wound dressing comprising a backing layer 2 of microporous liquid-impermeable polyurethane foam, such as ESTANE 5714F (Registered Trade Mark). The backing layer is permeable to water vapor, but impermeable to wound exudate and microorganisms.

The backing layer 2 is coated with a substantially continuous layer 3 of pressure-sensitive polyurethane adhesive. An absorbent island 4 is adhered to a central region of the adhesive-coated backing sheet 2.

The absorbent island 4 comprises an absorbent layer 5 of hydrophilic polyurethane foam prepared as described in EP-A-0541391 and having a basis weight of about 350g/m² and a thickness of about 1.5 mm.

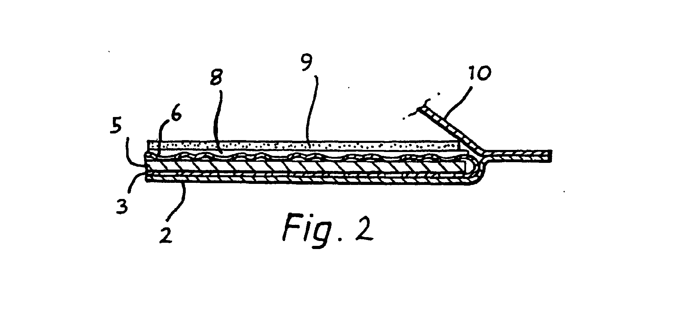

A barrier layer extends over the absorbent layer 5 and is wrapped partially around the absorbent layer 5, and the edges 7 of the barrier layer are adhered to the backing layer 2 behind the absorbent layer 5 by the adhesive 3. This can be seen more clearly in Figure 2. The barrier layer consists of a support layer 6 of vacuum mesh-perforated ethylene methyl acrylate (EMA) film coated with an occlusive layer 8 of poly(methyl methacrylate/methyl acrylate) pH-sensitive polymer.

Referring to Figure 2, the barrier layer presents a continuous top surface to the wound This surface is coated with a layer of hydrogel 9 applied by spraying. The hydrogel 9 has a dry basis weight of 30g/m² and consists of bovine gelatin cross-linked with glutaraldehyde or formaldehyde. The hydrogel layer 9 contains about 10% by weight on a dry weight basis of a sodium hydrogen phosphate/sodium dihydrogen phosphate buffer.

The wound facing surface of the dressing shown in Figure 1 is protected by two silicone-coated release papers 10,11. The dressing is packaged in a microorganism-impermeable pouch (not shown), and sterilised using gamma radiation.

In use, the dressing 1 is removed from the package, the release papers 10,11 are removed, and the dressing is adhered to the skin around the wound by the adhesive layer 3, with the barrier layer and hydrogel 9 in contact with the wound to provide a sterile and absorbent dressing. The hydrogel 9 absorbs wound exudate and maintains a moist and slightly acid-buffered environment at the wound surface. As more exudate is produced, it neutralises the hydrogel layer and the pH-sensitive polymer 8 on the barrier layer dissolves, allowing the excess exudate to escape through the support layer 6 into the absorbent layer 5. In this way, the dressing can provide an improved wound healing environment for an extended time on both high- and low- exuding wounds. Furthermore, the dissolution of the barrier layer can trigger the release of antimicrobial active agents from the absorbent layer into the wound in response to increased exudate production by infected wounds.

The above embodiment has been described by way of example only.

## Claims

1. A layered wound dressing material comprising:
a wound facing hydrogel layer; and
a barrier layer, wherein the barrier layer comprises a pH-sensitive material that is substantially insoluble in water at 25°C and pH4, but substantially soluble in water at 25°C and pH8, and wherein the hydrogel layer comprises a weak water-soluble acid buffer system.

2. A layered wound dressing material according to claim 1, wherein the barrier layer is in contact with the wound facing hydrogel layer.

3. A layered wound dressing material according to claim 1 or 2, further comprising an absorbent layer separated from the wound facing hydrogel layer by the barrier layer.

4. A layered wound dressing material according to claim 3, further comprising a liquid-impermeable backing layer over the absorbent layer opposite the barrier layer.

5. A layered wound dressing material according to claim 4, wherein the backing layer is adhesive-coated and extends beyond at least one edge of the wound facing hydrogel layer and the barrier layer.

6. A layered wound dressing material according to any preceding claim, wherein the hydrogel layer has a dry basis weight of from 10 to 200g/m².

7. A layered wound dressing material according to any preceding claim, wherein the hydrogel layer comprises a hydrogel material selected from polyurethanes, carboxymethyl cellulose, hydroxyethyl cellulose, hydroxypropyl methyl cellulose, modified acrylamides and mixtures thereof.

8. A layered wound dressing material according to any of claims 1 to 6, wherein the hydrogel layer comprises a gel formed from vinyl alcohols, vinyl esters, vinyl ethers and carboxy vinyl monomers, meth(acrylic) acid, acrylamide, N-vinyl pyrrolidone, acylamidopropane sulphonic acid, pluronic (block polyethylene glycol, block polypropylene glycol)polystyrene maleic acid, NN-dimethylacrylamide, diacetone acrylamide or acryloyl morpholine.

9. A layered wound dressing material according to any preceding claim, wherein the hydrogel layer comprises an active therapeutic agent or an antimicrobial agent.

10. A layered wound dressing material according to claim 9, wherein the hydrogel layer comprises a silver compound.

11. A layered wound dressing material according to any preceding claim, wherein the weak water-soluble acid buffer system is present in an amount of at least 0.2 mmol/g.

12. A layered wound dressing material according to claim 11, wherein the weak water-soluble acid buffer system is present in an amount of from 0.3 to 2.0 mmol/g.

13. A layered wound dressing material according to any one of preceding claim, wherein the acid component(s) of the water-soluble acid buffer has/have a pKₐ in the range of from 3.5 to 6.5.

14. A layered wound dressing material according to any preceding claim, wherein the hydrogel layer has an acid buffering capacity of at least 0.05 mmol/gram dry weight of the hydrogel layer.

15. A layered wound dressing material according to claim 14, wherein the hydrogel layer has an acid buffering capacity of at least 0.1 mmol/g.

16. A layered wound dressing material according to any preceding claim, wherein the weak water-soluble system is selected from the group consisting of water-soluble organic carboxylic acids and dihydrogen phosphate salts, and mixtures thereof in combination with one or more conjugate bases thereof.

17. A layered wound dressing material according to claim 16, wherein said organic carboxylic adds are selected from the group consisting of C₂-C₆ alkanoic acids, benzoic acid, substituted benzoic acids, citric acid, tartaric acid, malic acid, lactic add, succinic acid and ascorbic acid.

18. A layered wound dressing material according to any preceding claim, wherein said weak water-soluble buffer system comprises sodium hydrogen phosphate/sodium dihydrogen phosphate.

19. A layered wound dressing material according to any preceding claim, wherein a 10%w/v dispersion (dry weight basis) of said hydrogel material in water provides an equilibrium pH at 25°C of from 4.5 to 6.5.

20. A layered wound dressing material according to any preceding claim, wherein the water-soluble acid and the conjugate base of said buffer system are both solids when anhydrous at 25°C.

21. A layered wound dressing material according to any preceding claim, wherein the pH-sensitive material comprises a polymer selected from the group consisting of cellulose derivatives, starch derivatives, pectins, polyacrylates, polyvinyl acetate phthalate, and mixtures thereof.

22. A layered wound dressing material according to any preceding claim, wherein the pH-sensitive material comprises a cellulose derivative selected from cellulose acetate phthalate, cellulose acetate trimellitate, hydroxypropyl methyl cellulose, hydroxypropyl methyl cellulose acetate succinate, carboxymethyl ethyl cellulose, oxidised regenerated cellulose, and mixtures thereof.

23. A layered wound dressing material according to any preceding claim, wherein the pH-sensitive material comprises a polyacrylate selected from the copolymers of methacrylic acid with methyl methacrylate.

24. A layered wound dressing material according to any preceding claim, wherein the barrier layer comprises a substantially continuous film of the pH-sensitive material.

25. A layered wound dressing material according to any preceding claim, wherein the barrier layer comprises a liquid-permeable support layer having the pH-sensitive material applied thereto.

26. A layered wound dressing material according to any preceding claim, further comprising a reservoir of antimicrobial material on the side of the barrier layer opposite to the hydrogel layer.

27. A layered wound dressing material according to claim 26, wherein the antimicrobial material is dispersed in an absorbent layer.

28. A wound dressing comprising a layered wound dressing material according to any one of claims 1 to 27.

## Patentansprüche

1. Geschichtetes Wundverbandsmaterial, welches umfasst:
eine der Wunde zugewandte Hydrogelschicht; und
eine Barriereschicht, wobei die Barriereschicht ein pH-empfindliches Material umfasst, das in Wasser bei 25°C und pH 4 im wesentlichen unlöslich ist, jedoch in Wasser bei 25°C und pH 8 im wesentlichen löslich ist, und wobei die Hydrogelschicht ein schwaches wasserlösliches Säurepuffersystem umfasst.

2. Geschichtetes Wundverbandsmaterial nach Anspruch 1, wobei die Barriereschicht in Kontakt mit der der Wunde zugewandten Hydrogelschicht ist.

3. Geschichtetes Wundverbandsmaterial nach Anspruch 1 oder 2, weiter umfassend eine Absorbensschicht, die von der der Wunde zugewandten Hydrogelschicht durch die Barriereschicht getrennt ist.

4. Geschichtetes Wundverbandsmaterial nach Anspruch 3, weiter umfassend eine flüssigkeitsimpermeable Rückschicht über der Absorbensschicht gegenüberliegend der Barriereschicht.

5. Geschichtetes Wundverbandsmaterial nach Anspruch 4, wobei die Rückschicht klebstoffbeschichtet ist und sich über wenigstens einen Rand der der Wunde zugewandten Hydrogelschicht und der Barriereschicht erstreckt.

6. Geschichtetes Wundverbandsmaterial nach einem der vorangehenden Ansprüche, wobei die Hydrogelschicht ein Trockenflächengewicht von 10 bis 200 g/m² aufweist.

7. Geschichtetes Wundverbandsmaterial nach einem der vorangehenden Ansprüche, wobei das Hydrogel ein Hydrogelmaterial umfasst, das ausgewählt ist aus Polyurethanen, Carboxymethylcellulose, Hydroxyethylcellulose, Hydroxypropylmethylcellulose, modifizierten Acrylamiden und Mischungen derselben.

8. Geschichtetes Wundverbandsmaterial nach einem der Ansprüche 1 bis 6, wobei die Hydrogelschicht ein Gel umfasst, das aus Vinylalkoholen, Vinylestern, Vinylethem und Carboxyvinylmonomeren, Meth(acryl)säure, Acrylamid, N-Vinylpyrrolidon, Acylamidopropansulfonsäure, Pluronic-(Blockpolyethylenglykol, Blockpolypropylenglykol)-Polystyrolmaleinsäure, NN-Dimethylacrylamid, Diacetonacrylamid oder Acryloylmorpholin gebildet ist.

9. Geschichtetes Wundverbandsmaterial nach einem der vorangehenden Ansprüche, wobei die Hydrogelschicht ein aktives therapeutisches Agens oder ein antimikrobielles Agens umfasst.

10. Geschichtetes Wundverbandsmaterial nach Anspruch 9, wobei die Hydrogelschicht eine Silberverbindung umfasst.

11. Geschichtetes Wundverbandsmaterial nach einem der vorangehenden Ansprüche, wobei das schwache wasserlösliche Säurepuffersystem in einer Menge von wenigstens 0,2 mmol/g vorhanden ist.

12. Geschichtetes Wundverbandsmaterial nach Anspruch 11, wobei das schwache wasserlösliche Säurepuffersystem in einer Menge von 0,3 bis 2,0 mmol/g vorhanden ist.

13. Geschichtetes Wundverbandsmaterial nach einem der vorangehenden Ansprüche, wobei die Säurekomponente(n) des wasserlöslichen Säurepuffers einen pKₐ im Bereich von 3,5 bis 6,5 aufweist bzw. aufweisen.

14. Geschichtetes Wundverbandsmaterial nach einem der vorangehenden Ansprüche, wobei die Hydrogelschicht eine Säurepufferkapazität von wenigstens 0,05 mmol/g Trockengewicht der Hydrogelschicht aufweist.

15. Geschichtetes Wundverbandsmaterial nach Anspruch 14, wobei die Hydrogelschicht eine Säurepufferkapazität von wenigstens 0,1 mmol/g aufweist.

16. Geschichtetes Wundverbandsmaterial nach einem der vorangehenden Ansprüche, wobei das schwache wasserlösliche System ausgewählt ist aus der Gruppe bestehend aus wasserlöslichen organischen Carbonsäuren und Dihydrogenphosphatsalzen und Mischungen derselben in Kombination mit einer oder mehreren konjugierten Basen derselben.

17. Geschichtetes Wundverbandsmaterial nach Anspruch 16, wobei die organischen Carbonsäuren ausgewählt sind aus der Gruppe bestehend aus C₂-C₆-Alkansäuren, Benzoesäure, substituierten Benzoesäuren, Zitronensäure, Weinsäure, Äpfelsäure, Milchsäure, Bernsteinsäure und Ascorbinsäure.

18. Geschichtetes Wundverbandsmaterial nach einem der vorangehenden Ansprüche, wobei das schwache wasserlösliche Puffersystem Natriumhydrogenphosphat/Natriumdihydrogenphosphat umfasst.

19. Geschichtetes Wundverbandsmaterial nach einem der vorangehenden Ansprüche, wobei eine 10% w/v Dispersion (Trockengewichtsbasis) des Hydrogelmaterials in Wasser einen Gleichgewichts-pH-Wert bei 25°C von 4,5 bis 6,5 bereitstellt.

20. Geschichtetes Wundverbandsmaterial nach einem der vorangehenden Ansprüche, wobei die wasserlösliche Säure und die konjugierte Base des Puffersystems beides Feststoffe sind, wenn sie bei 25°C wasserfrei sind.

21. Geschichtetes Wundverbandsmaterial nach einem der vorangehenden Ansprüche, wobei das pH-empfindliche Material ein Polymer umfasst, das ausgewählt ist aus der Gruppe bestehend aus Cellulosederivaten, Stärkederivaten, Pektinen, Polyacrylaten, Polyvinylacetatphthalat und Mischungen derselben.

22. Geschichtetes Wundverbandsmaterial nach einem der vorangehenden Ansprüche, wobei das pH-empfindliche Material ein Cellulosederivat umfasst, das ausgewählt ist aus Celluloseacetatphthalat, Celluloseacetattrimellitat, Hydroxypropylmethylcellulose, Hydroxypropylmethylcelluloseacetatsuccinat, Carboxymethylethylcellulose, oxidierter regenerierter Cellulose und Mischungen derselben.

23. Geschichtetes Wundverbandsmaterial nach einem der vorangehenden Ansprüche, wobei das pH-empfindliche Material ein Polyacrylat umfasst, das ausgewählt ist aus den Copolymeren von Methacrylsäure mit Methylmethacrylat.

24. Geschichtetes Wundverbandsmaterial nach einem der vorangehenden Ansprüche, wobei die Barriereschicht einen im wesentlichen kontinuierlichen Film des pH-empfindlichen Materials umfasst.

25. Geschichtetes Wundverbandsmaterial nach einem der vorangehenden Ansprüche, wobei die Barriereschicht eine flüssigkeitspermeable Trägerschicht mit dem darauf aufgetragenen pH-empfindlichen Material umfasst.

26. Geschichtetes Wundverbandsmaterial nach einem der vorangehenden Ansprüche, weiter umfassend ein Reservoir von antimikrobiellem Material auf der Seite der Barriereschicht gegenüberliegend der Hydrogelschicht.

27. Geschichtetes Wundverbandsmaterial nach Anspruch 26, wobei das antimikrobielle Material in einer Absorbensschicht dispergiert ist.

28. Wundverband umfassend ein geschichtetes Wundverbandsmaterial nach einem der Ansprüche 1 bis 27.

## Revendications

1. Matériau multicouches pour le pansement des plaies comprenant:
une couche d'hydrogel située face à la plaie ; et
une couche barrière, la couche barrière comprenant une substance sensible au pH, essentiellement insoluble dans l'eau à 25°C et à pH 4, mais essentiellement soluble dans l'eau à 25°C et à pH 8, et la couche d'hydrogel comprenant un système tampon acide faible soluble dans l'eau.

2. Matériau multicouches pour le pansement des plaies selon la revendication 1, dans lequel la couche barrière est en contact avec la couche d'hydrogel située face à la plaie.

3. Matériau multicouches pour le pansement des plaies selon les revendications 1 ou 2, comprenant, en outre, une couche absorbante séparée de la couche d'hydrogel située face à la plaie par la couche barrière.

4. Matériau multicouches pour le pansement des plaies selon la revendication 3, comprenant, en outre, une couche de renfort imperméable aux liquides située au-dessus de la couche absorbante à l'opposé de la couche barrière.

5. Matériau multicouches pour le pansement des plaies selon la revendication 4, dans lequel la couche de renfort est revêtue d'un adhésif et se prolonge au-delà d'au moins un bord de la couche d'hydrogel située face à la plaie et de la couche barrière.

6. Matériau multicouches pour le pansement des plaies selon l'une quelconque des revendications précédentes, dans lequel la couche d'hydrogel présente un poids à sec de base de 10 à 200 g/m².

7. Matériau multicouches pour le pansement des plaies selon l'une quelconque des revendications précédentes, dans lequel la couche d'hydrogel comprend un matériau de type hydrogel choisi parmi les polyuréthanes, la carboxyméthylcellulose, l'hydroxyéthylcellulose, l'hydroxypropylméthylcellulose, les acrylamides modifiés et les mélanges de ceux-ci.

8. Matériau multicouches pour le pansement des plaies selon l'une quelconque des revendications 1 à 6, dans lequel la couche d'hydrogel comprend un gel constitué d'alcools vinyliques, d'esters vinyliques, d'éthers vinyliques et de monomères carboxyvinyliques, d'acide méth-(acrylique), d'acrylamide, de N-vinylpyrrolidone, d'acide acylamidopropanesulfonique, d'acide pluronique (polyéthylèneglycol à blocs, polypropylèneglycol à blocs) polystyrène maléique, de NN-diméthylacrylamide, de diacétone acrylamide ou d'acryloylmorpholine.

9. Matériau multicouches pour le pansement des plaies selon l'une quelconque des revendications précédentes, dans lequel la couche d'hydrogel comprend un agent thérapeutique actif ou un agent antimicrobien.

10. Matériau multicouches pour le pansement des plaies selon la revendication 9, dans lequel la couche d'hydrogel comprend un composé à base d'argent.

11. Matériau multicouches pour le pansement des plaies selon l'une quelconque des revendications précédentes, dans lequel le système tampon acide faible soluble dans l'eau est présent à hauteur d'au moins 0,2 mmol/g.

12. Matériau multicouches pour le pansement des plaies selon la revendication 11, dans lequel le système tampon acide faible soluble dans l'eau est présent à hauteur de 0,3 à 2,0 mmol/g.

13. Matériau multicouches pour le pansement des plaies selon l'une quelconque des revendications précédentes, dans lequel le(s) composant(s) acide(s) du tampon acide soluble dans l'eau présente(nt) un pKa se situant dans un intervalle de 3,5 à 6,5.

14. Matériau multicouches pour le pansement des plaies selon l'une quelconque des revendications précédentes, dans lequel la couche d'hydrogel présente une capacité tampon acide d'au moins 0,05 mmol/gramme de poids à sec de la couche d'hydrogel.

15. Matériau multicouches pour le pansement des plaies selon la revendication 14, dans lequel la couche d'hydrogel présente une capacité tampon acide d'au moins 0,1 mmol/g.

16. Matériau multicouches pour le pansement des plaies selon l'une quelconque des revendications précédentes, dans lequel le système soluble dans l'eau faible est choisi dans le groupe constitué des acides carboxyliques organiques et des sels de dihydrogène phosphate solubles dans l'eau, ainsi que des mélanges de ceux-ci, une ou plusieurs bases leur étant conjuguées.

17. Matériau multicouches pour le pansement des plaies selon la revendication 16, dans lequel lesdits acides carboxyliques organiques sont choisis dans le groupe constitué des acides alcanoïques en C₂-C₆, de l'acide benzoïque, des acides benzoïques substitués, de l'acide citrique, de l'acide tartrique, de l'acide malique, de l'acide lactique, de l'acide succinique et de l'acide ascorbique.

18. Matériau multicouches pour le pansement des plaies selon l'une quelconque des revendications précédentes, dans lequel ledit système tampon faible soluble dans l'eau comprend de l'hydrogène phosphate de sodium/dihydrogène phosphate de sodium.

19. Matériau multicouches pour le pansement des plaies selon l'une quelconque des revendications précédentes, dans lequel une dispersion à 10 % poids/volume (sur la base du poids à sec) dudit matériau hydrogel dans de l'eau donne un pH à l'équilibre de 4,5 à 6,5 à 25°C.

20. Matériau multicouches pour le pansement des plaies selon l'une quelconque des revendications précédentes, dans lequel l'acide hydrosoluble et la base conjuguée dudit système tampon se présentent tous deux sous la forme d'un solide à l'état anhydre à 25°C.

21. Matériau multicouches pour le pansement des plaies selon l'une quelconque des revendications précédentes, dans lequel la substance sensible au pH comprend un polymère choisi dans le groupe constitué des dérivés de la cellulose, des dérivés de l'amidon, des pectines, des polyacrylates, de l'acétate phtalate de polyvinyle et des mélanges de ceux-ci.

22. Matériau multicouches pour le pansement des plaies selon l'une quelconque des revendications précédentes, dans lequel la substance sensible au pH comprend un dérivé de la cellulose choisi parmi l'acétate phtalate de cellulose, l'acétate trimellitate de cellulose, l'hydroxypropylméthylcellulose, l'acétate succinate d'hydroxypropylméthylcellulose, la carboxyméthyléthylcellulose, la cellulose régénérée oxydée et les mélanges de ceux-ci.

23. Matériau multicouches pour le pansement des plaies selon l'une quelconque des revendications précédentes, dans lequel la substance sensible au pH comprend un polyacrylate choisi parmi les copolymères de l'acide méthacrylique avec le méthacrylate de méthyle.

24. Matériau multicouches pour le pansement des plaies selon l'une quelconque des revendications précédentes, dans lequel la couche barrière comprend un film essentiellement continu constitué de la substance sensible au pH.

25. Matériau multicouches pour le pansement des plaies selon l'une quelconque des revendications précédentes, dans lequel la couche barrière comprend une couche de renfort perméable aux liquides sur laquelle est appliquée la substance sensible au pH.

26. Matériau multicouches pour le pansement des plaies selon l'une quelconque des revendications précédentes, comprenant, en outre, un réservoir de substance antimicrobienne situé sur le côté de la couche barrière à l'opposé de la couche d'hydrogel.

27. Matériau multicouches pour le pansement des plaies selon la revendication 26, dans lequel la substance antimicrobienne est dispersée dans une couche absorbante.

28. Pansement pour plaies comprenant un matériau multicouches pour le pansement des plaies selon l'une quelconque des revendications 1 à 27.
